(19) Europäisches Patentamt
European Patent Office
Office européen des brevets

(11) Veröffentlichungsnummer: **0 048 827**
**A1**

(12) # EUROPÄISCHE PATENTANMELDUNG

(21) Anmeldenummer: **81106720.6**

(22) Anmeldetag: **28.08.81**

(51) Int. Cl.³: **C 07 D 233/60,** A 61 K 31/415 // C07D233/61

(30) Priorität: **09.09.80 DE 3033918**

(43) Veröffentlichungstag der Anmeldung: **07.04.82 Patentblatt 82/14**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Reiser, Wolf, Dr., Klebitzweg 17, D-5600 Wuppertal 1 (DE)**
Erfinder: **Elbe, Hans-Ludwig, Dr., Dasnöckel 59, D-5600 Wuppertal 11 (DE)**
Erfinder: **Büchel, Karl Heinz, Prof. Dr., Dabringhausener Strasse 42, D-5093 Burscheid (DE)**
Erfinder: **Plempel, Manfred, Dr., Pahlkestrasse 5, D-5600 Wuppertal 1 (DE)**

(54) **Substituierte 2,4-Dichlorphenyl-imidazol-vinyl-ketone, Verfahren zu ihrer Herstellung sowie antimikrobielle Mittel, die diese Stoffe enthalten.**

(57) Substituierte 2,4-Dichlorphenyl-imidazolyl-vinyl-ketone der allgemeinen Formel

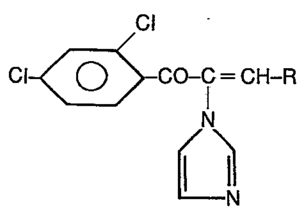

(I)

in welcher

R für Methyl, Ethyl, Isopropyl, Alkyl mit mehr als 3 Kohlenstoffatomen, gegebenenfalls substituiertes Cycloalkyl und Cycloalkenyl, gegebenenfalls substituiertes Cycloalkylalkyl und Cycloalkenylalkyl, gegebenenfalls substituiertes Alkenyl und Alkinyl, sowie gegebenenfalls im Alkylteil und im Phenylteil substituiertes Phenylalkyl steht, und deren physiologisch verträglichen Säureadditions-Salze haben gute antimykotische Eigenschaften und eignen sich als Wirkstoffe für Arzneimittel.

EP 0 048 827 A1

0048827

BAYER AKTIENGESELLSCHAFT 5090 Leverkusen, Bayerwerk
Zentralbereich Si/W
Patente, Marken und Lizenzen Ib

Substituierte 2,4-Dichlorphenyl-imidazol-vinyl-ketone, Verfahren zu ihrer Herstellung sowie antimikrobielle Mittel, die diese Stoffe enthalten.

Die vorliegende Erfindung betrifft neue substituierte 2,4-Dichlorphenyl-imidazolyl-vinyl-ketone, mehrere Verfahren zu ihrer Herstellung sowie ihre Verwendung als antimikrobielle Mittel, insbesondere als Antimykotika.

Es ist bereits bekannt geworden, daß bestimmte 1-Phenyl-2-imidazolyl-4,4-dimethyl-1-penten-3-one, wie z.B. das 1-(2-Chlorphenyl 2-(imidazol-1-yl)-4,4-dimethyl-1-penten-3-on, gute fungizide Eigenschaften besitzen (vergleiche die japanische Patentanmeldung J5 3130 661). Die Wirkung dieser Verbindungen gegen humanpathogene Pilze ist jedoch nicht immer voll befriedigend.

Es wurden neue substituierte 2,4-Dichlorphenyl-imidazolyl-vinyl-ketone der allgemeinen Formel

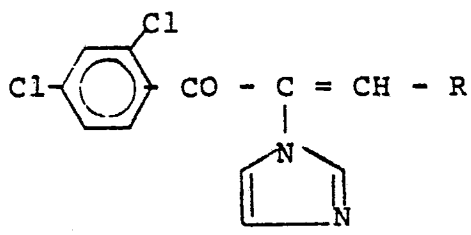

(I)

in welcher

R für Methyl, Ethyl, Isopropyl, Alkyl mit mehr als 3 Kohlenstoffatomen, gegebenenfalls substituiertes Cycloalkyl und Cycloalkenyl, gegebenenfalls substituiertes Cycloalkylalkyl und Cycloalkenylalkyl, gegebenenfalls substituiertes Alkenyl und Alkinyl, sowie gegebenenfalls im Alkylteil und im Phenylteil substituiertes Phenylalkyl steht,

und deren physiologisch verträglichen Säureadditions-Salze gefunden.

Die Verbindungen der Formel (I) können in zwei geometrischen Isomerenformen (E- und Z-Form) vorliegen, je nach Anordnung der Gruppen, die an die Doppelbindung gebunden sind; vorzugsweise fallen sie in einem wechselnden E,Z-Isomerenverhältnis an. Die vorliegende Erfindung betrifft sowohl die einzelnen Isomeren als auch die Isomeren-Gemische.

Weiterhin wurde gefunden, daß man die substituierten 2,4-Dichlorphenyl-imidazolyl-vinyl-ketone der Formel (I) erhält, wenn man

a) Ketoenamine der Formel

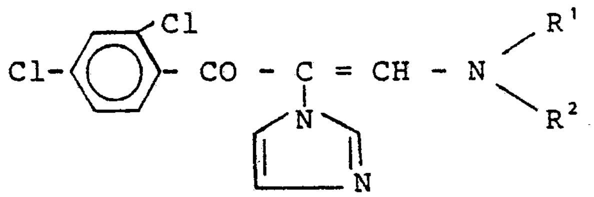

(II)

in welcher

$R^1$ und $R^2$ gleich oder verschieden sind und für Alkyl stehen,

mit magnesium-organischen Verbindungen der Formel

Hal - Mg - R (III)

in welcher

R die oben angegebene Bedeutung hat und
Hal für Halogen steht,

in Gegenwart eines Lösunsgmittels und gegebenenfalls in Gegenwart eines Katalysators umsetzt, oder

b) 2,4-Dichlorphenacyl-imidazol der Formel

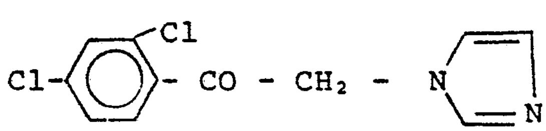

(IV)

mit Aldehyden der Formel

$$O = CH - R \qquad (V)$$

in welcher

R die oben angegebene Bedeutung hat,

in Gegenwart eines Lösungsmittels und in Gegenwart eines Katalysators umsetzt.

An die so erhaltenen Verbindungen der Formel (I) kann gegebenenfalls anschließend eine Säure addiert werden.

Die Verbindungen der Formel (I) können weiterhin an der Keto-Gruppe in üblicher und bekannter Weise derivatisiert werden, z.B. zu entsprechenden Oxim-, Oximether-, Ketal-, Hydrazon-Derivaten und ähnliche.

Die neuen substituierten 2,4-Dichlorphenyl-imidazolyl-vinyl-ketone der Formel (I) weisen starke antimykotische Eigenschaften auf. Dabei zeigen überraschenderweise die erfindungsgemäßen Verbindungen eine allgemein bessere Wirksamkeit als die aus dem Stand der Technik bekannten 1-Phenyl-2-imidazolyl-4,4-dimethyl-1-penten-3-one, wie z.B. das 1-(2-Chlorphenyl)-2-(imidazol-1-yl)-4,4-dimethyl-1-penten-3-on, welches chemisch naheliegende Verbindungen sind. Die erfindungsgemäßen Stoffe stellen somit eine Bereicherung der Pharmazie dar.

Die erfindungsgemäß substituierten 2,4-Dichlorphenyl-imidazolyl-vinyl-ketone sind durch die Formel (I) allgemein definiert. In dieser Formel steht R vorzugsweise für Methyl, Ethyl, Isopropyl, sowie für geradkettiges oder verzweigtes Alkyl mit 4 bis 29, insbesondere bis 18, Kohlenstoffatomen; für gegebenenfalls durch Alkyl mit 1 bis 4 Kohlenstoffatomen substituiertes Cycloalkyl und Cycloalkenyl mit jeweils 5 bis 7 Kohlenstoffatomen; für gegebenenfalls durch Alkyl mit 1 bis 4 Kohlenstoffatomen substituiertes Cycloalkylalkyl und Cycloalkenyl mit jeweils 5 bis 7 Kohlenstoffatomen im Cycloalkyl- bzw. Cycloalkenyl-Teil und jeweils 1 bis 6 Kohlenstoffatomen im geradkettigen oder verzweigten Alkyl-Teil; für gegebenenfalls substituiertes geradkettiges oder verzweigtes Alkenyl und Alkinyl mit jeweils bis zu 6 Kohlenstoffatomen, wobei als Substituenten genannt seien: Hydroxy, Alkoxy mit 1 bis 4 Kohlenstoffatomen, sowie Phenyl, das gegebenenfalls substituiert sein kann durch Halogen und Alkyl mit 1 bis 4 Kohlenstoffatomen; sowie für gegebenenfalls substituiertes Phenylalkyl mit 1 bis 4 Kohlenstoffatomen im Alkylteil, wie insbesondere Benzyl und 1-Phenyl-eth-1-yl, wobei als Phenylsubstituenten vorzugsweise infrage kommen: Halogen, Alkyl mit 1 bis 4 Kohlenstoffatomen, sowie Halogenalkyl mit 1 bis 2 Kohlenstoffatomen und 1 bis 5 gleichen oder verschiedenen Halogenatomen, wie insbesondere Fluor- und Chloratomen, und wobei als Alkylsubstituienten vorzugsweise infrage kommen: Cyano, Hydroxycarbonyl und Alkoxycarbonyl mit 1 bis 4 Kohlenstoffatomen im Alkylteil.

Besonders bevorzugt sind diejenigen Verbindungen der Formel (I), in denen R für gegebenenfalls durch Methyl und Ethyl substituiertes Cyclohexyl, Cyclohexenyl, Cyclopentyl, Cyclopentenyl, Cyclohexyl-methyl, Cyclohexyl-ethyl, 2-Cyclohexyl-prop-1-yl, Cyclohexenyl-methyl, Cyclohexenyl-ethyl oder 2-Cyclohexenyl-prop-1-yl steht; für Methacryl, Trimethylvinyl, Acetylenyl, Hydroxyacetylenyl, Methoxyacetylenyl,

Phenacetylenyl, Chlorphenylacetylenyl, Propargyl, Hydroxypropargyl, Methoxypropargyl, Phenylpropargyl oder Chlorphenylpropargyl steht; sowie für gegebenenfalls im Phenylteil durch Fluor, Chlor und Methyl substituiertes Benzyl oder 1-Phenyl-eth-1-yl steht, wobei die Methyl- bzw. Ethyl-Gruppe außerdem substituiert sein kann durch Cyano, Hydroxy- oder Methoxycarbonyl.

Im einzelnen seien außer den bei den Herstellungsbeispielen genannten Verbindungen die folgenden Verbindungen der allgemeinen Formel (I) genannt:

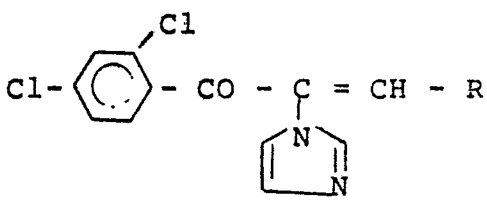

(I)

mit R = $-C_7H_{15}-n$

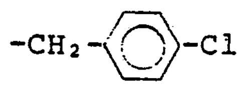

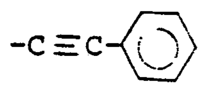

$-C(CH_3)_3$

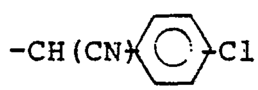

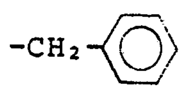

$-CH_2-C(CH_3)=CH_2$

$-C_4H_9-n$

Verwendet man 1-(2,4-Dichlorphenyl)-3-dimethylamin-2-(imidazol-1-yl)-2-propen-1-on und Ethylmagnesiumbromid als Ausgangsstoffe, so kann der Reaktionsablauf durch das folgende Formelschema wiedergegeben werden (Verfahren a):

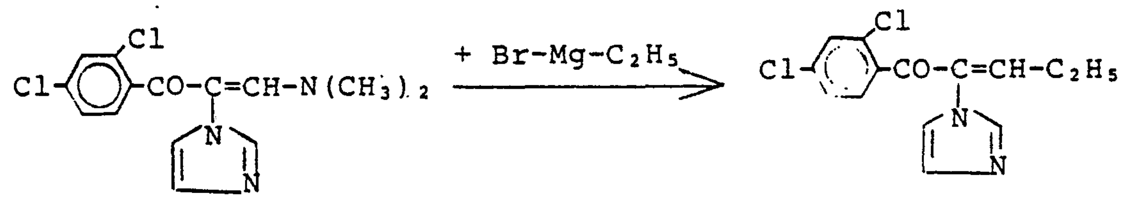


Verwendet man beispielsweise α-(Imidazol-1-yl)-2,4-dichloracetophenon und Cyclohexancarbaldehyd als Ausgangsstoffe, so kann der Reaktionsablauf durch das folgende Formelschema wiedergegeben werden (Verfahren b):

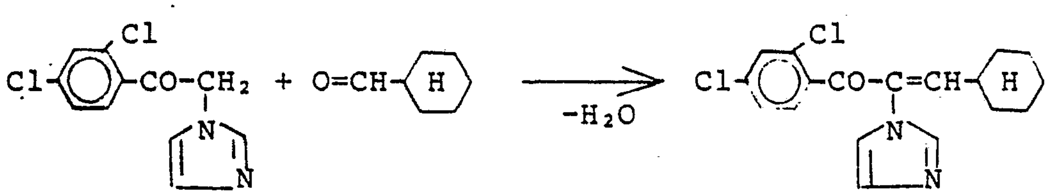


Die als Ausgangsstoffe für das erfindungsgemäße Verfahren (a) zu verwendenden Ketoenamine sind durch die Formel(II) allgemein definiert. In dieser Formel sind $R^1$ und $R^2$ gleich oder verschieden und stehen vorzugsweise für Alkyl mit 1 bis [illegible] Kohlenstoffatomen, insbesondere Methyl.

Die Ketoenamine der Formel (II) sind noch nicht bekannt; sie sind jedoch Gegenstand einer älteren eigenen Anmeldung, die noch nicht veröffentlicht ist (vergleiche die deutsche Patentanmeldung P 30 00 643.0 vom 10.1.80 [LeA 20 084]). Die Ketoenamine der Formel (II) können nach den dort beschriebenen Verfahren erhalten werden, indem man 2,4-Dichlorphenacyl-imidazol der Formel (IV) mit Amidacetalen bzw. Aminalestern der Formeln

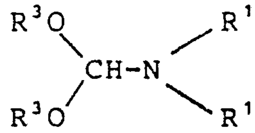

(VI a)

bzw.

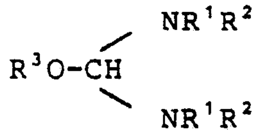

(VI b)

in welchen

$R^1$ und $R^2$ die oben angegebene Bedeutung haben und

$R^3$ für Alkyl mit 1 bis 4 Kohlenstoffatomen steht,

in an sich bekannter Art und Weise in Gengenwart eines inerten organischen Lösungsmittels, wie beispielsweise aromatische Kohlenwasserstoffe und wie insbesondere ein Ueberschuß an eingesetztem Amidacetal bzw. Aminalester der Formeln (VIa) bzw. (VIb), in der Siedehitze umsetzt (vergleiche hierzu auch Chem.Ber. 101, 41-50 (1968); J.Org.Chem. 43, 4248-50 (1978) sowie die Herstellungsbeispiele).

Die Amidacetale und Aminalester der Formeln (VIa) bzw. (VI b) sind allgemein bekannte Verbindungen der organischen Chemie (vergleiche z.B. Chem.Ber. 101, 41-50 (1968) und J.Org.Chem. 43, 4248-50 (1978)).

Die außerdem für die erfindungsgemäße Umsetzung gemäß Verfahren (a) als Ausgangsstoffe zu verwendenden magnesium-organischen Verbindungen sind durch die Formel (III) allgemein definiert. In dieser Formel steht R vorzugsweise für diejenigen Reste, die bereits im Zusammenhang mit der Beschreibung der erfinudngsgemäßen Stoffe der Formel (I) vorzugsweise für diese Substituenten genannt wurde. Hal steht vorzugsweise für Chlor oder Brom.

Die magnesium-organischen Verbindungen der Formel (III) sind allgemein bekannte Verbindungen der organischen Chemie.

Das als Ausgangsstoff für das erfindungsgemäße Verfahren (b) zu verwendende 2,4-Dichlorphenacyl-imidazol der Formel (IV) ist bekannt (vergleiche DE-OS 26 10 022) und wird erhalten, indem man das entsprechende Brom(Chlor)Keton in üblicher Weise in Gegenwart eines Säurebinders mit Imidazol umsetzt.

Die außerdem für das erfindungsgemäße Verfahren (b) als Ausgangsstoffe zu verwendenden Aldehyde sind durch die Formel (V) allgemein definiert. In dieser Formel steht <u>R</u> vorzugsweise für diejenigen Reste, die bereits im Zusammenhang mit der Beschreibung der erfindungsgemäßen Stoffe der Formel (I) für diesen Substituenten genannt werden.

Die Aldehyde der Formel (V) sind allgemein bekannte Verbindungen der organischen Chemie.

Als Lösungsmittel kommen für die erfindungsgemäße Umsetzung gemäß Verfahren (a) vorzugsweise inerte organische Lösungsmittel in reiner Form oder im Gemisch infrage. Hierzu gehören vorzugsweise Ether, wie Diethylether, Methylethylether, Tetrahydrofuran oder Dioxan, aliphatische und aromatische Kohlenwasserstoffe, wie insbesondere Benzol, Toluol und Xylol, sowie Hexamethylphosphorsäuretriamid.

Die Reaktionstemperaturen können bei der Durchführung des erfindungsgemäßen Verfahrens(a) in einem größeren Bereich variiert werden. Im allgemeinen arbeitet man zwischen -50°C und + 150°C, vorzugsweise zwischen -20°C und + 120°C.

Die erfindungsgemäße Umsetzung gemäß Verfahren (a) kann in Gegenwart eines Inertgases, wie z.B. Stickstoff oder Helium, durchgeführt werden.

Bei der Durchführung des erfindungsgemäßen Verfahrens (a) setzt man auf 1 Mol Ketoneamin der Formel (II) vorzugsweise 1 bis 1,5 Mol an magnesium-organischer Verbindung der Formel (III) ein. Die Isolierung der Verbindungen der Formel (I) erfolgt in üblicher Weise.

Als Lösungsmittel kommen für das erfindungsgemäße Verfahren (b) vorzugsweise inerte organische Lösungsmittel infrage. Hierzu gehören vorzugsweise Alkohole, wie Methanol und Ethanol; Ether, wie Tetrahydrofuran und Dioxan; aliphatische und cycloaliphatische Kohlenwasserstoffe, wie Hexan und Cyclohexan; aromatische Kohlenwasserstoffe, wie Benzol, Toluol und Cumol; halogenierte aliphatische und aromatische Kohlenwasserstoffe, wie Methylenchlorid, Tetrachlorkohlenstoff, Chloroform, Chlorbenzol und Dichlorbenzol.

Das erfindungsgemäße Verfahren (b) wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen, wie Pyridin und Piperidin; sowie insbesondere Piperidinacetat.



BAD ORIGINAL

Die Reaktionstemperaturen können bei der Durchführung des erfindungsgemäßen Verfahrens (b) in einem größeren Bereich variiert werden. Im allgemeinen arbeitet man zwischen 20 und 160°C, vorzugsweise bei der Siedetemperatur des jeweiligen Lösungsmittels.

Bei der Durchführung des erfindungsgemäßen Verfahrens (b) setzt man auf 1 Mol Imidazol-keton der Formel (IV) 1 bis 1,5 Mol Aldehyd der Formel (V) und katalytische bis o,2 molare Mengen an Katalysator ein. Die Isolierung der Verbindungen der Formel (I) erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren, wie z.B. p-Touolsulfonsäure und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.



BAD ORIGINAL

Die erfindungsgemäßen Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bisphatische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung bekämpfbaren Keime dar, sondern hat nur erläuternden Charakter.

Als Indikatonsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:
Alle Dermatomykosen und Systemmykosen, insbesondere solche, die durch die obengenannten Erreger hervorgerufen werden.



BAD ORIGINAL

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabellen, Dragges, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabeletten, Dragees, Kapseln, Pillen, Granulate, Suppositorien, Lösungen, Suspensionen und Emulsionen,

Pasten, Salben, Gele, Cremes, Lotions, Puder und Sprays genannt.

Tabeletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchtehaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Den Tabeletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können benen dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Püder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler oder Emulgatoren, z.B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Oele, insbesondere Baumwollsaatöl, Erdnußöl, Meiskeimöl, Olivenöl, Ricinusöl und Sesaöl, Glycerin, Glycerinformal, Tetrahydrofurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isosterarylalkohole, Polyoxyäthylensorbit- und Sorbitanester, mikrokristalline Cellulose, Aluminiummetahydroxi, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süßmittel, z.B. Sacharin enthalten.

Die therapeutisch wirksamen Verbindungen sollten in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen nach weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/ oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankungen, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

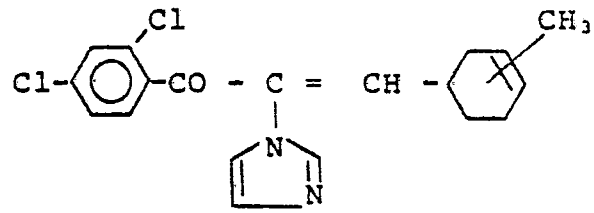

(Verfahren b)

19,1g (0,075 Mol) 2,4-Dichlorphenacyl-imidazol, 9,3g (0,075 Mol) Methylcyclohexen-aldehyd, 1,83 g (20 Mol %) Benzoesäure und 0,96g (15 Mol %) Piperidin werden in eienm Gemisch aus 75 ml Cyclohexan und 50 ml Toluol aufgenommen und eine Stunde am Wasserabscheider unter Rückfluß erhitzt. Nach dem Abkühlen wird das Reaktionsgemisch mit verdünnter Natriumhydrogencarbonatlösung und zweimal mit Wasser gewaschen, die organische Phase wird abgetrennt, über Natriumsulfat getrocknet, filtriert und eingeengt. Das zurückbleibende Oel wird entgast. Man erhält 27g (99 % der Theorie) 1-(2,4-Dichlorphenyl)-2-(imidazol-1-yl)-3-(methylcyclohexen-3-yl)-2-propen-1-on als zähes Oel.

Beispiel 2

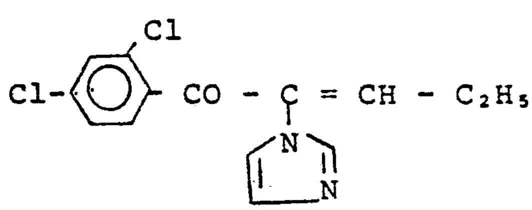

(Verfahren a)

10g (0,033 Mol) 1-(2,4-Dichlorphenyl)- 3-dimethylamino-2-(imidazol-1-yl)-2-propen-1-on werden in loo ml Tetrahydro-



BAD ORIGINAL

furan gelöst. Dazu tropft man bei -20°C eine Lösung von 5,5 g Ethylmagnesiumbromid in 50 ml Ether. Man läßt das Reaktionsgemisch innerhalb von 30 Minuten auf Raumtemperatur erwärmen und rührt 2 Stunden bei dieser Temperatur nach. Danach wird mit verdünnter Salzsäure versetzt, die organische Phase wird abgetrennt, mit Wasser gewaschen, über Natriumsulfat getrocknet und eingeengt. Der Rückstand wird säulenchromatographisch gereinigt. Man erhält 6g (62 % der Theorie) 1-(2,4-Dichlorphenyl)-2-(imidazol-1-yl)-2-penten-1-on als Oel vom Brechungsindex $n_D^{20}$= 1,4384.

Herstellung des Ausgangsproduktes

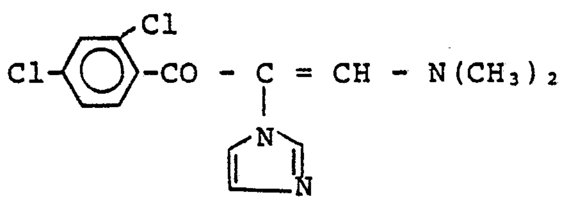

58g (0,227 Mol) 2,4-Dichlorphenacyl-imidazol und 33,4g (0,253 Mol) Bis-dimethylamino-methoxy-methan werden 2 Stunden bei Raumtemperatur gerührt. Anschließend wird so lange auf 50-60°C erwärmt, bis die Dimethylamin-Entwicklung beendet ist. Das abgekühlte Reaktionsgemisch wird mit heißem Diisopropylether gewaschen, der Rückstand in Methylenchlorid aufgenommen, mit Aktivkohle gereinigt und eingeengt. Man erhält 53,1g (76 % der Theorie) 1-(2,4-Dichlorphenyl)-3-dimethylamino-2-(imidazol-1-yl)-2-propen-1-on vom Schmelzpunkt 55°C.

Analog werden die folgenden Verbindungen der allgemeinen Formel (I)

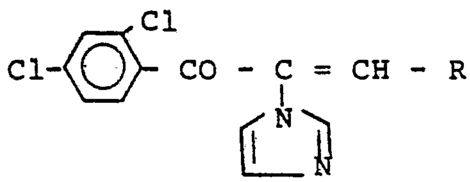 (I)

erhalten:

| Bsp.Nr. | R | physikal. Konstante |
|---|---|---|
| 3 | 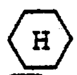 | zähfl.Oel |
| 4 | - 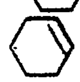 | zähfl.Oel |
| 5 | $-CH(C_2H_5)_2$ | zähfl.Oel |
| 6 | $-CH(CH_3)-C_2H_5$ | zähfl.Oel |
| 7 | $-CH(CH_3)-C_3H_7-n$ | zähfl.Oel |
| 8 | $-CH_2-CH(CH_3)-$ 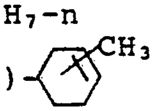 | zähfl.Oel |
| 9 | $-CH(C_2H_5)-C_4H_9-n$ | zähfl.Oel |
| 10 | $-C_6H_{13}-n$ | zähfl.Oel |
| 11 | $-CH(CH_3)-$ 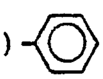 | zähfl.Oel |
| 12 | $-CH_3$ | zähfl.Oel |

Verwendungsbeispiele

In dem nachfolgenden Beispiel wird die nachstehend angegebene aus der japanischen Patentanmeldung 75 3130 661 bekannte Vergleichssubstanz eingesetzt:

(A) = 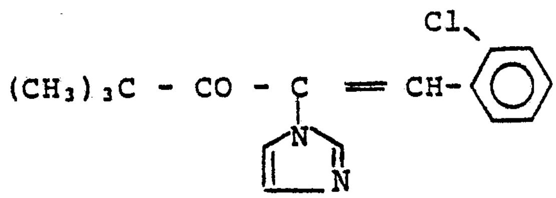

Beispiel A

Antimykotische in-vitro-Wirksamkeit

Versuchsbeschreibung:

Die in-vitro-Prüfung wurde im Reihenverdünnungstest mit Keiminokula von durchschnittlich 5 x 10 Keimen/ml Substrat durchgeführt. Als Nährmedium dienten

a) für Dermatophyten und Schimmelpilze:
Sabourand's milieu d'épresive

b) für Hefen:
Isotonic Sensitest-Broth von Oxid.

Die Bebrütungstemperatur betrug 28°C; Bebrütungsdauer war 24 Stunden bei Hefen und 96 Stunden bei Dermatophyten und Schimmelpilzen.

In diesem Test zeigen insbesondere die Beispiele 1,5,6, 7 und 9 eine bessere antimykotische Wirkung als die aus dem Stand der Technik bekannte Verbindung (A).

Patentansprüche

1. Substituierte 2,4-Dichlorphenyl-imidazolyl-vinyl-ketone der allgemeinen Formel (I)

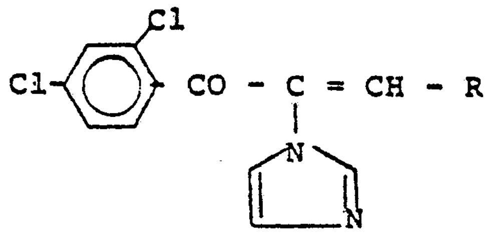

(I)

in welcher

R für Methyl, Ethyl, Isopropyl, Alkyl mit mehr als 3 Kohlenstoffatomen, gegebenenfalls substituiertes Cycloalkyl und Cycloalkenyl, gegebenenfalls substituiertes Cycloalkylalkyl und Cycloalkenylalkyl, gegebenenfalls substituiertes Alkenyl und Alkinyl, sowie gegebenenfalls im Alkylteil und im Phenylteil substituiertes Phenylalkyl steht,

und deren physiologisch verträglichen Säureadditions-Salze.

2. Substituierte 2,4-Dichlorphenyl-imidazolyl-vinyl-ketone der allgemeinen Formel (I) in Anspruch 1, in welcher

R vorzugsweise für Methyl, Ethyl, Isopropyl, sowie für geradkettiges oder verzweigtes Alkyl mit 4 bis 29, insbesondere bis 18, Kohlenstoffatomen; für gegebenenfalls durch Alkyl mit 1 bis 4 Kohlenstoffatomen substituiertes Cycloalkyl mit Cycloalkenyl mit jeweils 5 bis 7 Kohlenstoffatomen; für gegebenenfalls durch Alkyl mit 1 bis 4 Kohlenstoffatomen substituiertes Cycloalkylalkyl und Cycloalkenyl mit jeweils 5 bis 7 Kohlenstoffatomen im Cycloalkyl- bzw. Cycloalkenyl-Teil und jeweils 1 bis 6 Kohlenstoffatomen im geradkettigen oder verzweigten Alkyl-Teil; für gegebenenfalls substituiertes geradkettiges oder verzweigtes Alkenyl und Alkinyl mit jeweils bis zu 6 Kohlenstoffatomen, wobei als Substituenten genannt seien: Hydroxy, Alkoxy mit 1 bis 4 Kohlenstoffatomen, sowie Phenyl, das gegebenenfalls substituiert sein kann durch Halogen und Alkyl mit 1 bis 4 Kohlenstoffatomen; sowie für gegebenenfalls substituiertes Phenylalkyl mit 1 bis 4 Kohlenstoffatomen im Alkylteil, wie insbesondere Benzyl und 1-Phenyl-eth-1-yl, wobei als Phenylsubstituenten vorzugsweise infrage kommen: Halogen, Alkyl mit 1 bis 4 Kohlenstoffatomen, sowie Halogenalkyl mit 1 bis 2 Kohlenstoffatomen und 1 bis 5 gleichen oder verschiedenen Halogenatomen, wie insbesondere Fluor- und Chloratomen, und wobei als Alkylsubstituenten vorzugsweise infrage kommen: Cyano, Hydroxycarbonyl und Alkoxycarbonyl mit 1 bis 4 Kohlenstoffatomen im Alkylteil.

3. Substituierte 2,4-Dichlorphenyl-imidazolyl-vinylketone der allgemeinen Formel (I) in Anspruch 1

in welcher

R für gegebenenfalls durch Methyl und Ethyl substituiertes Cyclohexyl, Cyclohexenyl, Cyclopentyl, Cyclopentenyl, Cyclohexyl-methyl, Cyclohexyl-ethyl, 2-Cyclohexyl-prop-1-yl, Cyclohexenylmethyl, Cyclohexenyl-ethyl oder 2-Cyclohexenylprop-1-yl steht; für Methacryl, Trimethyl-vinyl, Acetylenyl, Hydroxyacetylenyl, Methoxyacetylenyl, Phenacetylenyl, Chlorphenylacetylenyl, Propargyl, Hydroxypropargyl, Methoxypropargyl, Phenylpropargyl oder Chlorphenylpropargyl steht; sowie für gegebenenfalls im Phenylteil durch Fluor, Chlor und Methyl substituiertes Benzyl oder 1-Phenyl-eth-1-yl steht, wobei die Methyl- bzw. Ethyl-Gruppe außerdem substituiert sein kann durch Cyano, Hydroxy- oder Methoxycarbonyl.

4. 1-(2,4-Dichlorphenyl)-2-(imidazol-1-yl)-3-(methylcyclohexen-3-yl)-2-propen-1-on.

5. 1-(2,4-Dichlorphenyl)-2-(imidazol-1-yl)-4-ethyl-2-hexen-1-on.

6. 1-(2,4-Dichlorphenyl)-2-(imidazol-1-yl)-4-ethyl-2-octen-1-on.

7. Verfahren zur Herstellung von substituierten 2,4-Dichlorphenyl-imidazolyl-vinyl-ketonen der Formel (I) in Anspruch 1, dadurch gekennzeichnet, daß man die substituierten 2,4-Dichlorphenyl-imidazolyl-vinyl-ketone der Formel (I) erhält, wenn man

a) Ketoenamine der Formel

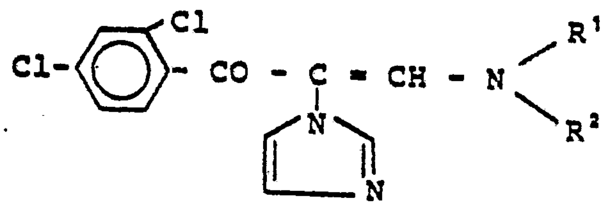

(II)

in welcher

$R^1$ und $R^2$ gleich oder verschieden sind und für Alkyl stehen,

mit magnesium-organischen Verbindungen der Formel

$$Hal - Mg - R \qquad (III)$$

in welcher

R die oben angegebene Bedeutung hat und
Hal für Halogen steht,

in Gegenwart eines Lösungsmittels und gegebenenfalls in Gegenwart eines Katalysators umsetzt, oder

b) 2,4-Dichlorphenacyl-imidazol der Formel

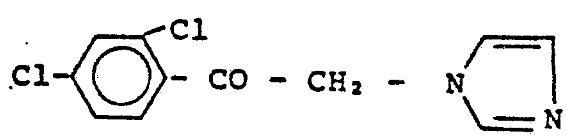

(IV)

mit Aldehyden der Formel

$$O = CH - R \qquad (V)$$

in welcher

R die oben angegebene Bedeutung hat,

in Gegenwart eines Lösungsmittels und in Gegenwart eines Katalysators umsetzt.

8. Arzneimittel, gekennzeichnet durch einen Gehalt an mindestens einem substituierten 2,4-Dichlorphenyl-imidazolyl-vinyl-keton gemäß Anspruch 1.

9. Verfahren zur Herstellung von antimykotischen Mitteln, dadurch gekennzeichnet, daß man substituierte 2,4-Dichlorphenyl-imidazolyl-vinylketone gemäß Anspruch 1 mit inerten, nichttoxischen, pharmazeutisch geeigneten Trägerstoffen vermischt.

10. Verfahren zur Behandlung von Mykosen, dadurch gekennzeichnet, daß man substituierte 2,4-Dichlorphenyl-imidazolyl-vinyl-ketone gemäß Anspruch 1 Menschen oder Tieren appliziert, die an Mykosen erkrankt sind.

0048827

Europäisches Patentamt

# EUROPÄISCHER RECHERCHENBERICHT

Nummer der Anmeldung
EP 81 10 6720

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | CHEMICAL ABSTRACTS, Band 94, Nr. 17, 27. April 1981, Seite 767, Nr. 139814c<br>Columbus, Ohio, U.S.A.<br>& JP - A - 80 111 477 (A.G. BAYER) 28-08-1980<br>* Zusammenfassung *<br>& EP - A - 0 032 239<br>-- | 1-10 | C 07 D 233/60<br>A 61 K 31/415//<br>C 07 D 233/61 |
| | GB - A - 1 504 352 (I.C.I.)<br>* Insgesamt *<br>-- | 1-10 | RECHERCHIERTE SACHGEBIETE (Int. Cl.³) |
| | US - A - 4 182 862 (HAK-FOON CHAN)<br>* Insgesamt *<br>-- | 1-10 | C 07 D 233/60 |
| E | EP - A - 0 032 239 (BAYER A.G.)<br>* Insgesamt *<br>---- | 1-10 | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 18-01-1982 | ALLARD |